# EUROPEAN PATENT SPECIFICATION

(11) **EP 2 368 485 B1**
(45) Date of publication and mention of the grant of the patent: **23.03.2016**
(21) Application number: 11152067.2
(22) Date of filing: 25.01.2011
(51) Int. Cl.: A61B 1/04, A61B 5/1455

(54) **Electronic endoscope system**
Elektronisches Endoskopsystem
Système endoscope électronique

(30) Priority: 26.03.2010 JP 2010071913
(43) Date of publication of application: 28.09.2011
(73) Proprietor: Fujifilm Corporation, Minato-ku Tokyo (JP)
(72) Inventor: Chun, Minkyung, Kanagawa-ken (JP)
(74) Representative: Klunker . Schmitt-Nilson . Hirsch

(56) References cited:
- EP-A2- 2 368 486
- JP-A- 2002 085 344
- US-A- 4 878 113
- US-A1- 2006 184 037
- US-A1- 2009 247 881

## Description

### BACKGROUND OF THE INVENTION

The present invention relates to an electronic endoscope system for acquiring information on a blood vessel from an image acquired by an electronic endoscope and displaying the acquired information.

In recent years, a number of diagnoses and treatments using electronic endoscopes have been made in the field of medicine. A typical electronic endoscope is equipped with an elongated insertion section that is inserted into a subject's body cavity. The insertion section has therein incorporated an imager such as a CCD at the tip thereof. The electronic endoscope is connected to a light source device, which emits light from the tip of the insertion section to illuminate the inside of a body cavity. With the inside of the body cavity illuminated by light, the subject tissue inside the body cavity is imaged by an imager provided at the tip of the insertion section. Images acquired by imaging undergoes various kinds of processing by a processor connected to the electronic endoscope before being displayed by a monitor. Thus, the electronic endoscope permits real-time observation of images showing the inside of the subject's body cavity and thus enables sure diagnoses.

The light source device uses a white light source such as a xenon lamp capable of emitting white broadband light whose wavelength ranges from a blue region to a red region. Use of white broadband light to illuminate the inside of a body cavity permits observing the whole subject tissue from the acquired images thereof. However, although images acquired by broadband light illumination permit generally observing the whole subject tissue, there are cases where such images fail to enable clear observation of subject tissues such as micro-blood vessels, deep blood vessels, pit patterns, and uneven surface profiles formed of recesses and bumps. As is known, such subject tissues may be made clearly observable when illuminated by narrowband light having a wavelength limited to a specific range. As is also known, image data obtained by illumination with narrowband light yields various kinds of information on a subject tissue such as oxygen saturation level in a blood vessel, which acquired information is converted into an image.

JP 2648494 B, for example, describes acquiring an oxygen saturation level image using narrowband light, giving two examples: three narrowband light IR1, IR2, and IR3 each having different wavelengths in near infrared range; and three narrowband light G1, G2, and G3 each having different wavelengths in visible light range. Both combinations contain narrowband light having a wavelength band with which blood hemoglobin exhibits a variation in degree of light absorption (absorbance) according to the oxygen saturation level thereof and narrowband light having a wavelength band with which such a variation is not observed. JP 2648494 B describes selecting two of the three signals corresponding to the three narrowband light having different wavelengths and detecting the differences among them to display an oxygen saturation level image in monochrome or in simulated color.

JP 3583731 B describes separating white light produced by a discharge lamp such as a xenon lamp through filters into three colors having different wavelengths, red, green, and blue, and illuminating a subject to acquire information on a tissue lying in a desired depth from a subject surface.

There exists prior art according to Article 54 (3) EPC, i. e. EP 2 368 486 A2, which discloses an electronic endoscope system, in which an image production means produces an oxygen saturation level image in which a blood vessel in a certain depth is intensified. An image display means of this endoscope system is adapted to display an image in which a blood vessel in a certain depth is represented.

US 2009/247881 discloses a device according to the preamble of claim 1.

### SUMMARY OF THE INVENTION

In recent years, there are demands for a system permitting diagnosis accompanied by simultaneous observation of blood vessel depth and oxygen saturation level. Because the reaching depth of light generally varies with wavelength, the combination of wavelengths appropriate for sensing the change in hemoglobin varies depending on the depth of a blood vessel to be observed. However, according to the method described in JP 2648494 B, the depth at which the oxygen saturation level can be measured must remain the same, making it impossible for a single device to measure the oxygen saturation level when the depth varies. The method described in JP 3583731 A permits acquisition of configuration information for a desired depth but is unable to acquire information on the oxygen saturation level of hemoglobin in a blood vessel.

An object of the present invention is to provide an electronic endoscope system capable of simultaneous acquisition of information on blood vessel depth and oxygen saturation level and simultaneous display of images related to oxygen saturation levels of blood vessels lying in different depths.

In order to attain the above objects, the invention provides an electronic endoscope system as defined in claim 1.

Preferably, the light source device emits at least two first light and at least one second light as the plurality of light having different wavelength bands, the at least two first light having wavelength bands whereby the magnitude of light absorption reverses between oxygenated hemoglobin and reduced hemoglobin in the blood vessel depending on the oxygen saturation level, the at least one narrowband light having a wavelength band whereby the absorbance coincides.

Preferably, the light source device emits narrowband light having a central wavelength ±10nm as the first and the second light.

Preferably, the electronic endoscope comprises a color image sensor that converts the received light into the imaging signal through photoelectric conversion, and wherein the light source device simultaneously emits narrowband light having central wavelengths corresponding to spectral sensitivities of respective color channels of the image sensor.

Preferably, the electronic endoscope comprises a monochromatic image sensor that converts the received light into the imaging signal through photoelectric conversion, and wherein the light source device emits narrowband light having a first central wavelength in such an emission order that the narrowband light having the first central wavelength is emitted between narrowband light having second and third central wavelengths that permit easier acquisition of characteristics information of the subject tissue than does the narrowband light having the first central wavelength.

Preferably, the light source device emits broadband light having a wavelength band of 470 nm to 700 nm as the first and the second light, and wherein the image producing means produces the oxygen saturation level image from an imaging signal of the narrowband light and an imaging signal of at least one color channel of the broadband light that have been aligned by the alignment means.

Preferably, the image producing means produces an oxygen saturation level image representing a distribution of an oxygen saturation level in a plurality of blood vessels lying in different depths, and wherein the image display means simultaneously displays oxygen saturation level images of the plurality of blood vessels lying in different depths.

Preferably, the image display means simultaneously displays oxygen saturation level images of the plurality of blood vessels lying in different depths as separate two-dimensional images.

Preferably, the image display means simultaneously displays oxygen saturation level images of the plurality of blood vessels lying in different depths as one three-dimensional image.

The present invention permits simultaneous acquisition of information on blood vessel depth and oxygen saturation level by switching between wavelengths of illumination light for illuminating a subject and display of an image related to an oxygen saturation level of a blood vessel lying in a given depth.

### BRIEF DESCRIPTION OF THE DRAWINGS

Fig. 1 is an external view of an electronic endoscope system according to an embodiment of the invention.
Fig. 2 is a block diagram illustrating an electric configuration of the electronic endoscope system of Fig. 1.
Fig. 3 is a graph illustrating spectral transmittances of red, green, and blue filters.
Fig. 4 is a conceptual view illustrating a case of imaging with a color CCD where a plurality of narrowband light corresponding to the spectral sensitivities of channels B, G, and R are simultaneously emitted.
Fig. 5A is a view for explaining operations of a CCD in a normal light image mode; Fig. 5B is a view for explaining operations of a CCD in a special light image mode.
Fig. 6 is a conceptual view of a case of imaging with a monochromatic CCD where seven kinds of narrowband light are sequentially emitted.
Fig. 7 is a graph illustrating an absorption coefficient of hemoglobin.
Fig. 8 is a graph illustrating a correlation between first and second luminance ratios S1/S3 and S2/S3 on the one hand and blood vessel depth and oxygen saturation level on the other hand.
Fig. 9A is a view for explaining how a coordinate point (X*, Y*) in a luminance coordinate system is obtained from the first and the second luminance ratios S1*/S3* and S2*/S3*; Fig. 9B is a view for explaining how a coordinate point (U*, V*) in a blood vessel information coordinate system corresponding to the coordinate point (X*, Y*) is obtained.
Fig. 10 is a graph illustrating a correlation between blood density and oxygen saturation level.
Fig. 11 is a block diagram illustrating a specific configuration inside an oxygen saturation level image producer.
Fig. 12 is a graph illustrating a two-color color circle corresponding to oxygen saturation level.
Fig. 13A is a graph illustrating a shading corresponding to blood vessel depth; Fig. 13B is a graph illustrating a two-color gradation corresponding to blood vessel depth.
Fig. 14 is a screen view of a monitor simultaneously showing the oxygen saturation levels of a superficial-layer blood vessel, an intermediate-layer blood vessel, and a deep-layer blood vessel.
Fig. 15 is a screen view of a monitor showing the oxygen saturation levels of a superficial-layer blood vessel, an intermediate-layer blood vessel, and a deep-layer blood vessel in a simulated three-dimensional display.
Fig. 16 is a flowchart of the operations of an electronic endoscope system for imaging the oxygen saturation levels of a superficial-layer blood vessel, an intermediate-layer blood vessel, and a deep-layer blood vessel.

### DETAILED DESCRIPTION OF THE INVENTION

The electronic endoscope system according to the present invention will be described in detail based on preferred embodiments illustrated in the attached drawings.

The electronic endoscope system according to the present invention simultaneously obtains the oxygen saturation levels of hemoglobin in blood vessels in different depths by switching between illumination wavelengths for imaging depending on the depth of a blood vessel to be imaged as measured from the surface of the subject tissue and simultaneously displays a plurality of images of oxygen saturation levels at different depths.

An embodiment described below uses seven kinds of narrowband light having different central wavelengths of 405, 445, 473, 532, 560, 650, and 800 nm as a light source for acquiring a distribution of oxygen saturation levels of blood vessels in different depths. In view of general characteristics of light that the reaching depth as measured from the surface of a subject tissue attained by light emitted from a light source increases with the wavelength, light having an increasingly longer wavelength is used for a blood vessel to be imaged as its depth increases.

Specifically, for a superficial-layer blood vessel (depth about 10 µm to 100 µm), an intermediate-layer blood vessel (depth about 100 µm to 500 µm), and a deep-layer blood vessel (depth about 500 µm to 2000 µm), a combination of narrowband light having three different wavelengths as follows is used:
Superficial-layer blood vessel: central wavelength 405, 445, and 473 nm
Intermediate-layer blood vessel: central wavelength 473, 532, and 560 nm
Deep-layer blood vessel: central wavelength 560, 650, and 800 nm

Any of the combinations uses two narrowband light having wavelength bands whereby the magnitude of light absorption (absorbance) reverses between oxygenated hemoglobin and reduced hemoglobin depending on the oxygen saturation level of blood hemoglobin and one narrowband light having a wavelength band whereby the absorbance is the same.

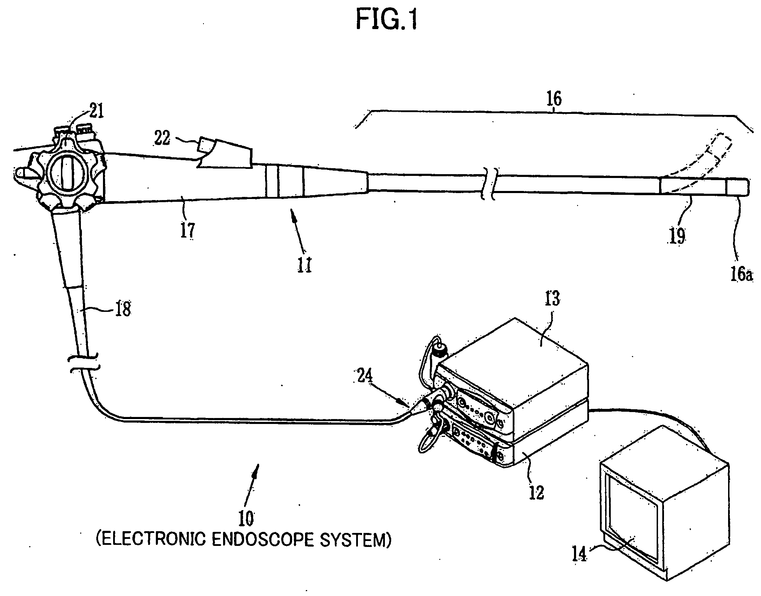

As illustrated in Fig. 1, an electronic endoscope system 10 of the invention comprises an electronic endoscope 11 for imaging the inside of a body cavity of a subject, a processor 12 for producing an image of a subject tissue in the body cavity containing a blood vessel region according to a signal acquired by imaging, a light source device 13 for supplying light for illuminating the inside of the body cavity, and a monitor 14 for displaying the image of the inside of the body cavity. The electronic endoscope 11 comprises a flexible insertion section 16 that is inserted into a body cavity, an operating section 17 provided at the base of the insertion section 16, and a universal cord 18 for connecting the operating section 17 to the processor 12 and the light source device 13.

The insertion section 16 has a bending portion 19 at the tip thereof comprising connected bending pieces. The bending portion 19 bends up and down, left and right in response to the operation of an angle knob 21 of the operating section 17. The bending portion 19 has at its tip a leading end portion 16a incorporating an optical system and other components for imaging the inside of a body cavity. The leading end portion 16a can be directed in a desired direction in the body cavity according to a bending operation of the bending portion 19. The operating section 17 has an insertion opening 22 in which a treatment tool or the like is inserted.

The universal cord 18 has a connector 24 provided on the side thereof leading to the processor 12 and the light source device 13. The connector 24 is a composite type connector composed of a communication connector and a light source connector. The electronic endoscope 11 removably connects to the processor 12 and the light source device 13 through the connector 24.

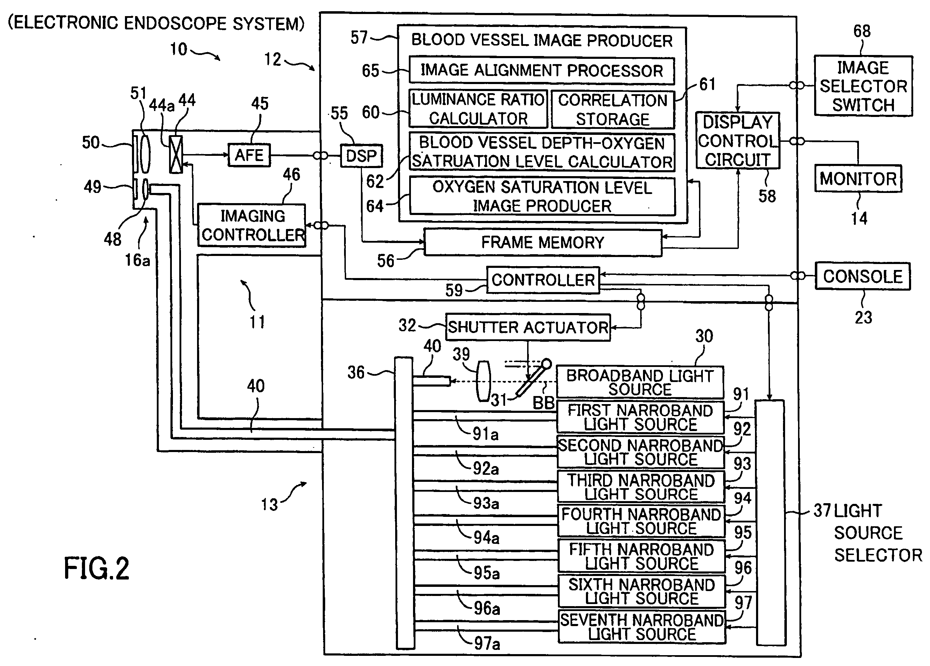

The light source device 13 emits a plurality of light having different wavelength bands and, as illustrated in Fig. 2, comprises a broadband light source 30, a shutter 31, a shutter actuator 32, first to seventh narrowband light sources 91 to 97, a coupler 36, and a light source selector 37.

The broadband light source 30 is a xenon lamp, a white LED, a Micro White (trademark) light source, or the like and produces broadband light BB having a wavelength ranging from a blue region to a red region (about 470 nm to 700 nm). The broadband light source 30 remains lighted at all times when the electronic endoscope 11 is in operation. The broadband light BB emitted from the broadband light source 30 is focused by a condenser lens 39 before entering a broadband optical fiber 40.

The shutter 31 is disposed between the broadband light source 30 and the condenser lens 39 so as to be movable between its inserted position where the shutter 31 is located on the optical path of the broadband light BB to block the broadband light BB and its retracted position where the shutter 31 is retracted from the inserted position to allow the broadband light BB to travel toward the condenser lens 39. The shutter actuator 32 is connected to a controller 59 in the processor to control the actuation of the shutter 31 according to an instruction from the controller 59.

Members related to broadband light such as the broadband light source 30, the shutter 31, the condenser lens 39, and the broadband optical fiber 40 are not the essential components according to this embodiment. The broadband light is used for imaging in a normal light image mode described later.

The first to the seventh narrowband light sources 91 to 97 are laser diodes or the like. The first narrowband light source 91 produces narrowband light having a wavelength limited to 445 nm +/- 10 nm, preferably 445 nm (referred to below as "first narrowband light N1"), the second narrowband light source 92 produces narrowband light having a wavelength limited to 473 nm +/- 10 nm, preferably 473 nm. (referred to below as "second narrowband light N2"), the third narrowband light source 93 produces narrowband light having a wavelength limited to 405 nm +/- 10 nm, preferably 405 nm (referred to below as "third narrowband light N3"), the fourth narrowband light source 94 produces narrowband light having a wavelength limited to 532 nm +/- 10 nm, preferably 532 nm (referred to below as "fourth narrowband light N4"), the fifth narrowband light source 95 produces narrowband light having a wavelength limited to 560 nm +/- 10 nm, preferably 560 nm (referred to below as "third narrowband light N5"), the sixth narrowband light source 96 produces narrowband light having a wavelength limited to 650 nm +/- 10 nm, preferably 650 nm (referred to below as "third narrowband light N6"), and the seventh narrowband light source 97 produces narrowband light having a wavelength limited to 800 nm +/- 10 nm, preferably 800 nm (referred to below as "seventh narrowband light N7"), . The first to the seventh narrowband light sources 91 to 97 are connected respectively to first to seventh narrowband optical fibers 91a to 97a, so that the first to the seventh narrowband light N1 to N7 emitted by the first to the seventh narrowband light sources 91 to 97 enter the first to the seventh narrowband optical fibers 91a to 97a.

The coupler 36 connects a light guide 43 in the electronic endoscope 11 to the broadband optical fiber 40 and the first to the seventh narrowband optical fibers 91a to 97a. Thus, the broadband light BB can enter the light guide 43 through the broadband optical fiber 40. The first to the seventh narrowband light N1 to N7 can enter the light guide 43 through the first to the seventh narrowband optical fibers 91a to 97a.

The light source selector 37 is connected to the controller 59 in the processor and turns on or off the first to the seventh narrowband light sources 91 to 97 according to an instruction given by the controller 59. According to this embodiment, when in the normal light image mode using the broadband light BB, the broadband light source 30 is switched on (where the shutter 31 is in its retracted position) to acquire normal light images while the first to the seventh narrowband light sources 91 to 97 are turned off. By contrast, when in the special light image mode using the first to the seventh narrowband light N1 to N7, the broadband light source 30 is turned off (where the shutter 31 is in the inserted position) while the first to the seventh narrowband light sources 91 to 97 are sequentially turned on to acquire special light images.

Specifically, the light source selector 37 first turns on the first narrowband light source 91. Then, imaging of the subject tissue is started with the first narrowband light N1 illuminating the inside of the body cavity. Upon completion of imaging, the controller 59 gives a light source switching instruction to turn off the first narrowband light source 91 and turn on the second narrowband light source 92. Thereafter, likewise the first to the seventh narrowband light sources 91 to 97 are sequentially turned on to carry out imaging. Upon completion of imaging with the seventh narrowband light N7 illuminating the inside of the body cavity, the seventh narrowband light source 97 is turned off.

According to this embodiment, the seven kinds of narrowband light having different central wavelengths (the first to the seventh narrowband light N1 to N7) illuminate the subject as they are sequentially switched between them every one-frame time to acquire 7 frames of images (seven still images) corresponding to their respective wavelengths, seven-frame time constituting one set.

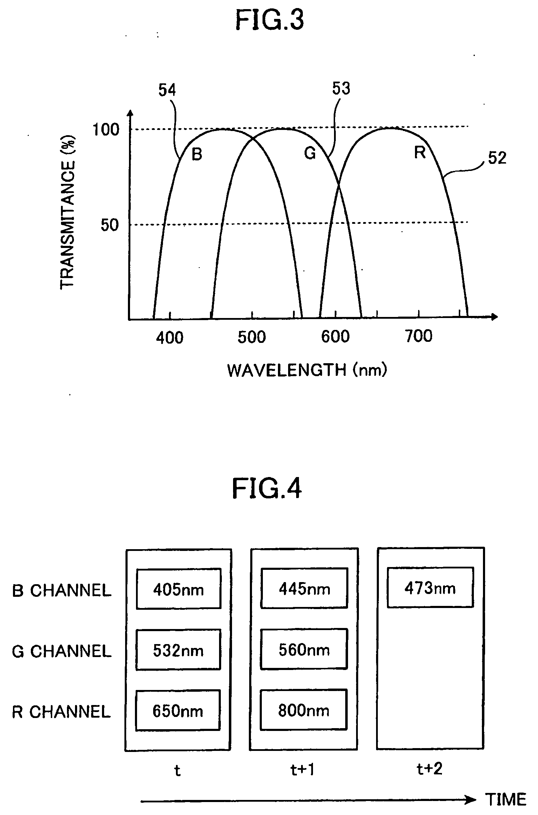

Preferably, the measuring (acquisition of images) is done in a short period of time in order to minimize the positional shifts (pixel shifts) between images acquired in the individual frames caused by the movement of the biological body (subject) and the like. Mention it later, when a color CCD having spectral sensitivities as illustrated in Fig. 3 is used, measuring three times (in three-frame time) by simultaneous illumination with a plurality of narrowband light respectively corresponding to the spectral sensitivities of the channels B, G, and R yields measurements corresponding to the seven kinds of narrowband light.

For example, as illustrated in Fig. 4, simultaneous illumination with the third narrowband light N3 having a central wavelength of 405 nm, the fourth narrowband light N4 having a central wavelength of 532 nm, and the sixth narrowband light N6 having a central wavelength of 650 nm in a first one-frame time t permits acquisition of the third narrowband signal composed of imaging signal B in the B channel, the fourth narrowband signal composed of imaging signal G in the G channel, and the sixth narrowband signal composed of imaging signal R in the R channel. Then, simultaneous illumination with the first narrowband light N1 having a central wavelength of 445 nm, the fifth narrowband light N5 having a central wavelength of 560 nm, and the seventh narrowband light N7 having a central wavelength of 800 nm in the next one-frame time t+1 permits acquisition of the first narrowband signal composed of imaging signal B in the B channel, the fifth narrowband signal composed of imaging signal G in the G channel, and the seventh narrowband signal composed of imaging signal R in the R channel. Illumination with the second narrowband light N2 having a central wavelength of 473 nm in the next one-frame time t+2 permits acquisition of the second narrowband signal composed of imaging signal B in the B channel.

Subsequently, the electronic endoscope 11 receives reflected light of the light sequentially illuminating a subject tissue in a body cavity containing a blood vessel and outputs an imaging signal corresponding to the wavelength band of the received light; the electronic endoscope 11 comprises the light guide 43, the CCD 44, an analog processing circuit 45 (AFE: analog front end) 45, and an imaging controller 46.

The light guide 43 is a large-diameter optical fiber, a bundle fiber, or the like having its light-receiving end inserted in the coupler 36 in the light source device, whereas its light emitting end is directed toward an illumination lens 48 located in the leading end portion 16a. The light emitted by the light source device 13 is guided by the light guide 43 and emitted toward the illumination lens 48. The light admitted in the illumination lens 48 passes through an illumination window 49 attached to the end face of the leading end portion 16a to enter the body cavity. The broadband light BB and the first to the seventh narrowband light N1 to N7 reflected by the inside of the body cavity pass through an observation window 50 attached to the end face of the leading end portion 16a to enter a condenser lens 51.

The CCD (image sensor) 44 receives the light from the condenser lens 51 with its imaging surface 44a, performs photoelectric conversion of the received light to accumulate a signal charge, and reads out the accumulated signal charge as an imaging signal. The CCD 44 according to this embodiment is a color CCD whose imaging surface 44a has arranged therein three colors of pixels, red (R) pixels (R channel), green (G) pixels (G channel), and blue (B) pixels (B channel), each provided with one of a red filter, a green filter, and a blue filter. The CCD 44 may be a monochromatic CCD.

As illustrated in Fig. 3, the red filters, the green filters, and the blue filters have spectral transmittances 52, 53, and 54, respectively. Among the light entering the condenser lens 51, the broadband light BB has a wavelength ranging from about 470 nm to 700 nm. Therefore, the red filters, the green filters, and the blue filters pass wavelength ranges respectively corresponding to their spectral transmittances 52, 53, 54 for the broadband light BB. Now, let imaging signal R be a signal photoelectrically converted by a red pixel, imaging signal G a signal photoelectrically converted by a green pixel, and imaging signal B a signal photoelectrically converted by a blue pixel. Then, the broadband light BB entering the CCD 44 gives a broadband imaging signal composed of the imaging signal R, the imaging signal G, and the imaging signal B.

Among the light entering the condenser lens 51, the first narrowband light N1, for example, has a wavelength of 440 nm +/-10 nm and therefore passes through only the blue filters. Accordingly, the first narrowband light N1 entering the CCD 44 yields a first narrowband imaging signal composed of an imaging signal B. The second narrowband light N2 has a wavelength of 470 nm +/- 10 nm and therefore passes through the blue and red filters. Accordingly, the second narrowband light N2 entering the CCD 44 yields a second narrowband imaging signal composed of an imaging signal B and an imaging signal G. The third narrowband light N3 has a wavelength of 400 nm +/- 10 nm and therefore passes through only the blue filters. Accordingly, the first narrowband light N3 entering the CCD 44 yields a third narrowband imaging signal composed of an imaging signal B. Thereafter, the fourth to the seventh narrowband light N4 to N7 likewise pass through the color filters corresponding to their respective wavelengths to yield fourth to seventh narrowband signals each composed of a corresponding imaging signal.

The AFE 45 comprises a correlated double sampling (CDS), an automatic gain control circuit (AGC), and an analog-to-digital converter (A/D) (none of them are shown). The CDS performs correlated double sampling of an imaging signal supplied from the CCD 44 to remove noise generated by actuation of the CCD 44. The AGC amplifies an imaging signal from which noise has been removed by the CDS. The analog-to-digital converter converts an imaging signal amplified by the AGC into a digital imaging signal having a given number of bits, which is applied to the processor 12.

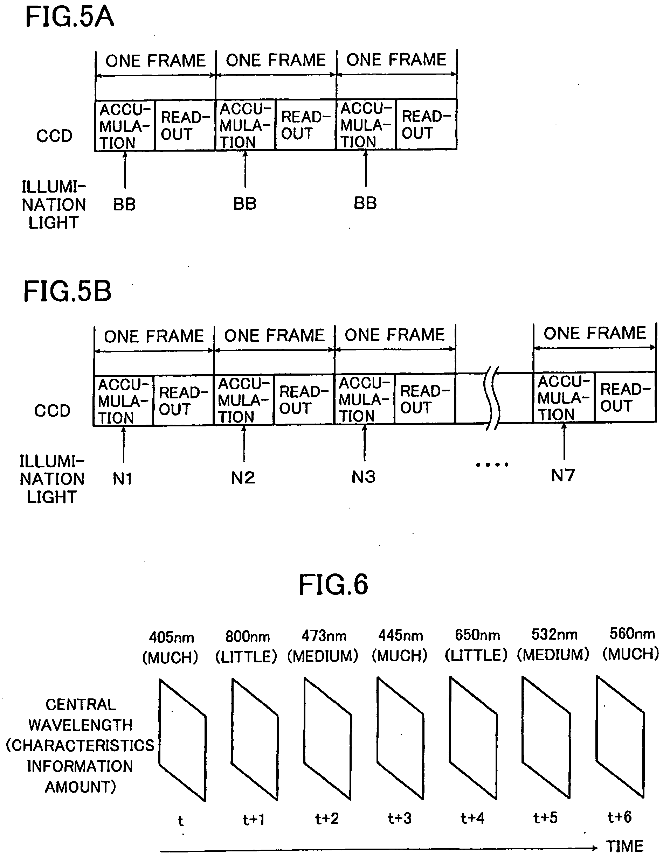

The imaging controller 46 is connected to the controller 59 in the processor 12 and sends a drive signal to the CCD 44 in response to an instruction given by the controller 59. The CCD 44 outputs an imaging signal to the AFE 45 at a given frame rate according to the drive signal from the imaging controller 46. According to this embodiment, when in the normal light image mode, a total of two operations are performed in an acquisition period of one-frame time as illustrated in Fig. 5A: a step of accumulating a signal charge through photoelectric conversion of the broadband light BB and a step of reading out the accumulated signal charge as a broadband imaging signal. These operations are repeated throughout the normal light image mode.

By contrast, when the mode is switched from the normal light image mode to the special light image mode, a total of two operations are first performed in an acquisition period of one-frame time as illustrated in Fig. 5B: a step of accumulating a signal charge through photoelectric conversion of the first narrowband light N1 and a step of reading out the accumulated signal charge as a first narrowband imaging signal. Upon completion of readout of the first narrowband imaging signal, in an acquisition period of one-frame time to follow, a step of accumulating a signal charge through photoelectric conversion of the second narrowband light N2 and a step of reading out the accumulated signal charge as a second narrowband imaging signal are performed. Upon completion of readout of the second narrowband imaging signal, in an acquisition period of one-frame time to follow, a step of accumulating a signal charge through photoelectric conversion of the third narrowband light N3 and a step of reading out the accumulated signal charge as a third narrowband imaging signal are performed. Thereafter, the accumulation and readout steps are likewise taken with the first to the seventh narrowband light N1 to N7 every one-frame time, for one set of seven-frame time. These operations are repeated throughout the special light image mode.

As illustrated in Fig. 2, the processor 12 comprises a digital signal processor 55 (DSP), a frame memory 56, a blood vessel image producer 57, and a display control circuit 58, all of these components being controlled by the controller 59. The DSP 55 performs color separation, color interpolation, white balance adjustment, gamma correction, and the like of the broadband imaging signal and the first to the seventh narrowband imaging signals outputted from the AFE 45 of the electronic endoscope to produce broadband image data and the first to the seventh narrowband image data. The frame memory 56 stores the broadband image data and the first to the seventh narrowband image data produced by the DSP 55. The broadband image data is color image data containing colors of red, green, and blue; the first to the seventh narrowband image data are color image data each containing only a corresponding color.

The blood vessel image producer 57 comprises an image alignment processor 65, a luminance ratio calculator 60, a correlation storage 61, a blood vessel depth-oxygen saturation level calculator 62, and an oxygen saturation level image producer 64.

The image alignment processor 65 aligns the images corresponding to the first to the seventh narrowband image data acquired in the special light image mode in one set composed of seven-frame time to correct positional shifts between the images acquired in the individual frames caused by the movement of the body tissue (subject) and the electronic endoscope. The first to the seventh narrowband image data that have undergone the alignment processing are stored in the frame memory 56. The alignment may be achieved by any of various methods as appropriate including known techniques. One may use a method, for example, whereby characteristics common to blood vessel images acquired at different times are detected, and a conversion matrix for correcting the positional shifts is produced and used to correct the positional shifts between the images acquired in the individual frames.

There arises a problem, however, that the characteristics information appropriate for alignment decreases as the measuring wavelength (wavelength of the illumination light at the time of imaging) increases. With a configuration using a color CCD having spectral sensitivities as illustrated in Fig. 3, this problem can be solved by, for example, obtaining a conversion matrix from B channel information (image data of imaging signals acquired by blue pixels) at 405 nm, 445 nm, and 473 nm where the characteristics can be readily acquired, and applying to the G channel and R channel the conversion matrix obtained from the B channel information that is measured at the same time as those channels.

When a monochromatic CCD is used, the order in which the measuring wavelengths are used is preferably given consideration to increase the accuracy with which the images acquired in the individual frames are aligned. More specifically, as illustrated in that drawing, the order in which the narrowband light are used for illumination is preferably determined in such a manner that the narrowband light having a central wavelength in a range making acquisition of characteristic information difficult is emitted between narrowband light each having a central wavelength in a range making acquisition of characteristics information easy to permit obtaining a conversion matrix of frames having only a small amount of characteristics information by complementation. According to this embodiment, the narrowband light of 800 nm and 473 nm are preferably emitted between the narrowband light of 405 nm and 445 nm; the narrowband light of 650 nm and 532 nm are preferably emitted between the narrowband light of 445 nm and 560 nm, as illustrated in Fig. 6.

Then, the luminance ratio calculator 60 determines a blood vessel region containing a blood vessel from, for example, the difference in luminance between a blood vessel portion and the other portion using the first to the seventh narrowband image data stored in the frame memory 56 that have undergone the alignment processing. The luminance ratio calculator 60 obtains, for example, a first luminance ratio S1/S3 between the first and the third narrowband image data and a second luminance ratio S2/S3 between the second and the third narrowband image data corresponding to a pixel at the same position in the blood vessel region. S1 is a luminance of a pixel of the first narrowband light image data, S2 a luminance of a pixel of the second narrowband light image data, and S3 a luminance of a pixel of the third narrowband light image data.

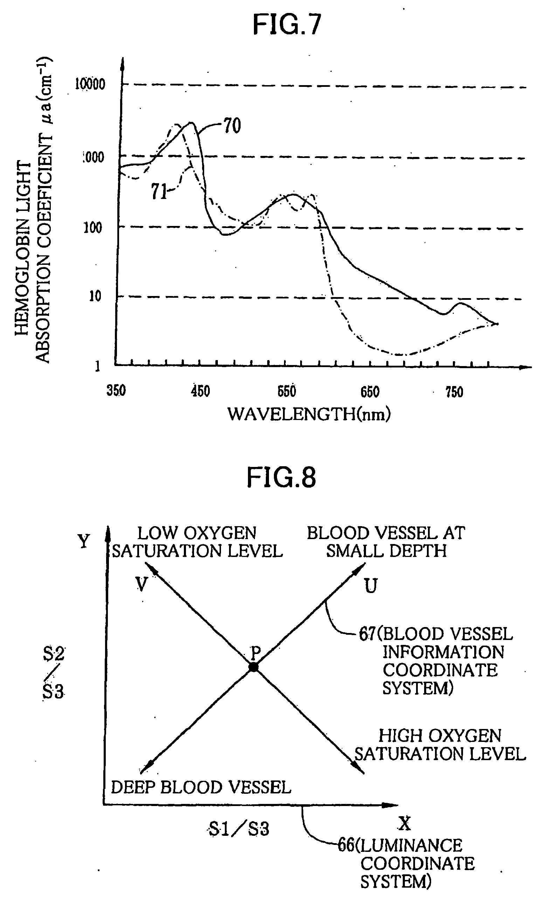

The correlation storage 61 stores, for example, a correlation between the first and the second luminance ratios S1/S3 and S2/S3 on the one hand and an oxygen saturation level in a blood vessel and a blood vessel depth on the other hand. That correlation is one where a blood vessel contains hemoglobin exhibiting light absorption coefficients as shown in Fig. 7 and is obtained by analyzing, for example, a number of the first to the seventh narrowband image data accumulated through diagnoses hitherto made. As illustrated in Fig. 7, the hemoglobins in a blood vessel have light absorptions characteristics having the light absorption coefficient µa changing according to the wavelength of light used for illumination. The light absorption coefficient µa indicates an absorbance or a degree of light absorption by hemoglobin and is a coefficient in an expression I₀exp(-µa×x) showing an attenuation of light by which hemoglobin was illuminated. In this expression, Io is the intensity of light emitted from the light source device to illuminate a subject tissue; x (cm) is a depth of a blood vessel inside the subject tissue.

A reduced hemoglobin 70 and an oxygenated hemoglobin 71 have different light absorption characteristics such that they have different absorbances except for the isosbestic points at which both exhibit the same absorbance (intersections of light absorption characteristics curves of hemoglobin 70 and 71 in Fig. 7). With a difference in absorbance, the luminance varies even when the same blood vessel is illuminated by light having the same intensity and the same wavelength. The luminance also varies when the illumination light has the same intensity but varies in wavelength because a difference in wavelength causes the light absorption coefficient µa to change. Further, with the same oxygen saturation level, a difference in wavelength causes a difference in absorption coefficient and also a difference in reaching depth into a mucus membrane. Therefore, using the property of light whose reaching depth varies with the wavelength permits obtaining correlation between luminance ratio and blood vessel depth.

As illustrated in Fig. 8, the correlation storage 61 stores a correlation in correspondence between the coordinate points in a luminance coordinate system 66 representing the first and the second luminance ratios S1/S3 and S2/S3 and the coordinate points in a blood vessel information coordinate system 67 representing oxygen saturation level and blood vessel depth. The luminance coordinate system 66 is an XY coordinate system, where the X axis shows the first luminance ratio S1/S3 and the Y axis shows the second luminance ratio S2/S3. The blood vessel information coordinate system 67 is a UV coordinate system provided on the luminance coordinate system 66, where the U axis shows the blood vessel depth and the V axis shows the oxygen saturation level. Because the blood vessel depth has a positive correlation with the luminance coordinate system 66, the U axis has a positive slope. The U axis shows that a blood vessel of interest is located at an increasingly smaller depth as a position on the U axis moves obliquely up rightward and that a blood vessel of interest is located at an increasingly greater depth as a position on the U axis moves obliquely down leftward. On the other hand, because the oxygen saturation level has a negative correlation with the luminance coordinate system 66, the V axis has a negative slope . The V axis shows that the oxygen saturation level is lower as a position on the V axis moves obliquely up leftward and that the oxygen saturation level is higher as a position on the V axis moves obliquely down rightward.

In the blood vessel information coordinate system 67, the U axis and the V axis cross each other at right angles at an intersection P. This is because the magnitude of absorbance reverses between illumination by the first narrowband light N1 and illumination by the second narrowband light N2. More specifically, as illustrated in Fig. 7, illumination by the first narrowband light N1 having a wavelength of 440 nm +/- 10 nm, for example, allows the light absorption coefficient of the reduced hemoglobin 70 to be greater than that of the oxygenated hemoglobin 71 having a high oxygen saturation level whereas illumination by the second narrowband light N2 having a wavelength of 470 nm +/- 10 nm allows the light absorption coefficient of the oxygenated hemoglobin 71 to be greater than that of the reduced hemoglobin 70 having a high oxygen saturation level, thus causing the magnitude of the absorbance to reverse. When narrowband light permitting no absorbance reversal are used in lieu of the first to the third narrowband light N1 to N3, the U axis and the V axis do not cross each other at right angles. With illumination provided by the first narrowband light N1 having a wavelength of 400 nm +/- 10 nm, the oxygenated hemoglobin and the reduced hemoglobin have substantially the same light absorption coefficient.

The blood vessel depth-oxygen saturation level calculator 62 determines an oxygen saturation level and a blood vessel depth corresponding to the first and the second luminance ratios S1/S3 and S2/S3 calculated by the luminance ratio calculator 60 based on the correlation stored in the correlation storage 61. Now, in the first and the second luminance ratios S1/S3 and S2/S3 calculated by the luminance ratio calculator 60, let S1*/S3* and S2*/S3* be the first luminance ratio and the second luminance ratio respectively for a given pixel in the blood vessel region.

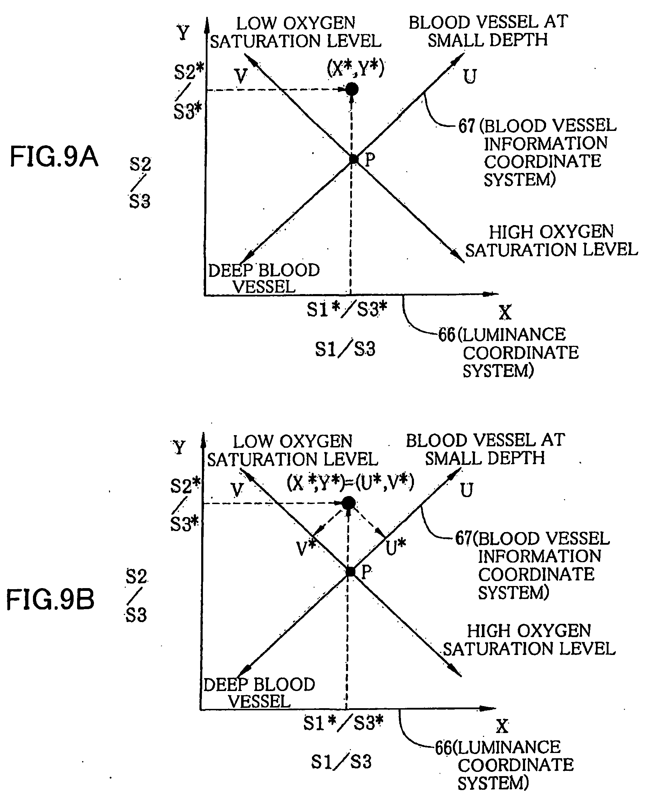

As illustrated in Fig. 9A, the blood vessel depth-oxygen saturation level calculator 62 determines a coordinate point (X*, Y*) corresponding to the first and the second luminance ratios S1*/S3* and S2*/S3* in the luminance coordinate system 66. Upon the coordinate point (X*, Y*) being determined, the blood vessel depth-oxygen saturation level calculator 62 determines a coordinate point (U*, V*) corresponding to the coordinate point (X*, Y*) in the blood vessel information coordinate system 67 as illustrated in Fig. 9B. Thus, blood vessel depth U* and oxygen saturation level V* are obtained for a given pixel in the blood region. The blood vessel depth is represented as numeric information such that the numeric value decreases with the decreasing blood vessel depth and increases with the increasing blood vessel depth. The oxygen saturation level is also represented in numeric information as is the blood vessel depth.

As described above, the blood vessel depth U* and the oxygen saturation level V* may be obtained using, for example, the first to the third narrowband image data corresponding to the first to the third narrowband light N1 to N3 having central wavelengths of 445 nm, 473 nm, and 405 nm. The above example is suitable for obtaining an oxygen saturation level of a superficial-layer blood vessel for which the narrowband light in those wavelength bands yields information on the blood vessel depth with a high resolution. On the other hand, with a wavelength of 473 nm or greater, the resolution of the blood vessel depth is lower than with a shorter wavelength, making it difficult to obtain the oxygen saturation level of blood vessels in the other layers if the same method is used as in the case of the superficial-layer blood vessel. Now, we will describe a method of calculating an oxygen saturation level appropriate for a combination of wavelengths of 473 nm or greater of narrowband light corresponding to intermediate-layer blood vessels and deep-layer blood vessels.

Now, let L1 be a narrowband light source producing light having a wavelength with which the absorbance does not vary with the oxygen saturation level of hemoglobin, L2 a narrowband light source producing light having a wavelength with which the absorbance increases as the oxygen saturation level increases, and L3 a narrowband light source producing light having a wavelength with which the absorbance decreases as the oxygen saturation level increases.

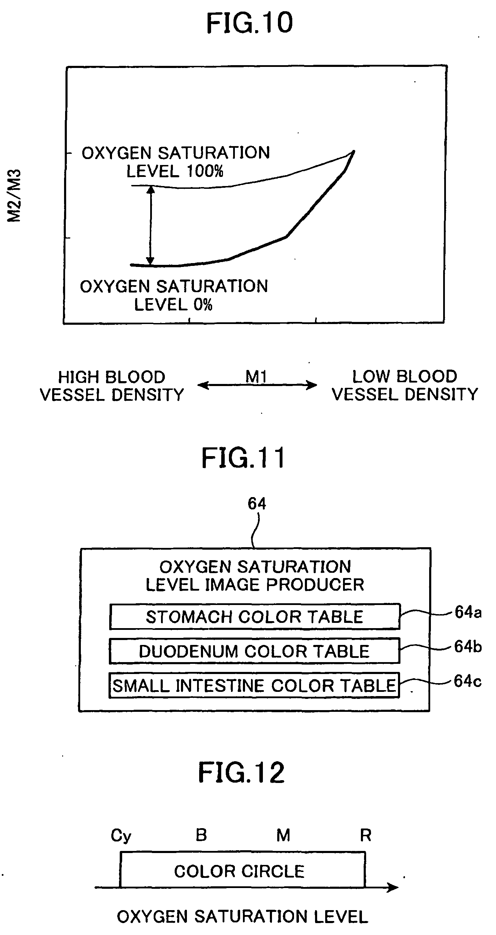

In the case of an intermediate-layer blood vessel, for example, L1 corresponds to the fourth narrowband light source 94 having a wavelength of 532 nm, L2 corresponds to the second narrowband light source 92 having a wavelength of 473 nm, and L3 corresponds to the fifth narrowband light source 95 having a wavelength of 560 nm. Let M1 to M3 be reflected signals (luminance of pixels in the narrowband image data) obtained by illuminating the subject with the light from the narrowband light sources L1 to L3. Then, the luminance M1 corresponds to the blood vessel density in the subject tissue, and the luminance ratio M2/M3 between M2 and M3 corresponds to the magnitude of the oxygen saturation level. Thus, the correlation between the luminance M1 and the luminance ratio M2/M3 exhibits a distribution as illustrated in Fig. 10. This distribution yields the oxygen saturation level V* in the intermediate-layer blood vessel.

In this case, the luminance ratio calculator 6C obtains a luminance M1 of the fourth narrowband image data and a luminance ratio M2/M3 of the second and the fifth narrowband image data for a pixel in the same position in the blood vessel region. The correlation storage 61 stores the correlation between the luminance M1 and the luminance ratio M2/M3 and the oxygen saturation level V* of the intermediate-layer blood vessel. The blood vessel depth-oxygen saturation level calculator 62 determines the oxygen saturation level V* corresponding to the luminance M1 and the luminance ratio M2/M3 calculated by the luminance ratio calculator 60 based on the correlation illustrated in Fig. 10 stored in the correlation storage 61. The oxygen saturation level of the deep-layer blood vessel may be likewise obtained as in the case of the intermediate-layer blood vessel.

Generally, the distribution illustrated in Fig. 10 changes into a non-linear line depending on the site of the subject and, therefore, needs to be previously obtained by measuring an actual body tissue or conducting a simulation of light propagation or the like. The oxygen saturation levels V* of the intermediate-layer blood vessel and the deep-layer blood vessel need not essentially be obtained.

As illustrated in Fig. 11, the oxygen saturation level image producer 64 comprises stomach color table 64a, a duodenum color table 64b, and a small intestine color table 64c, where the oxygen saturation level is assigned color information according to its magnitude. The color tables 64a to 64c can be switched between them by a switching operation on the console 23 to select a color table appropriate for a particular site to be observed. The stomach color table 64a contains color information adapted to the oxygen saturation level in the stomach; the duodenum color table 64b contains color information adapted to the oxygen saturation level in the duodenum; and the small intestine color table 64c contains color information adapted to the oxygen saturation level in the small intestine. The oxygen saturation level image producer 64 uses one of the color tables 64a to 64c selected by the console 23 to determine color information corresponding to the oxygen saturation level V* for the superficial-layer blood vessel, the intermediate-layer blood vessel, and the deep-layer blood vessel determined based on the blood vessel depth U* calculated by the blood vessel depth-oxygen saturation level calculator 62.

While this embodiment uses three kinds of color tables for stomach, duodenum, and small intestine, the kinds of color tables are not limited; for example, the color tables may further include one corresponding to a site of another subject tissue.

The color tables 64a to 64c in the oxygen saturation level image producer 64 are each represented by a two-color color circle of Cy (cyan) changing to R (red) as illustrated in Fig. 12. In Fig. 12, the color information represents oxygen saturation level in Cy when it is small, changing to B (blue) through M (magenta) to R (red) in this order as the oxygen saturation level increases.

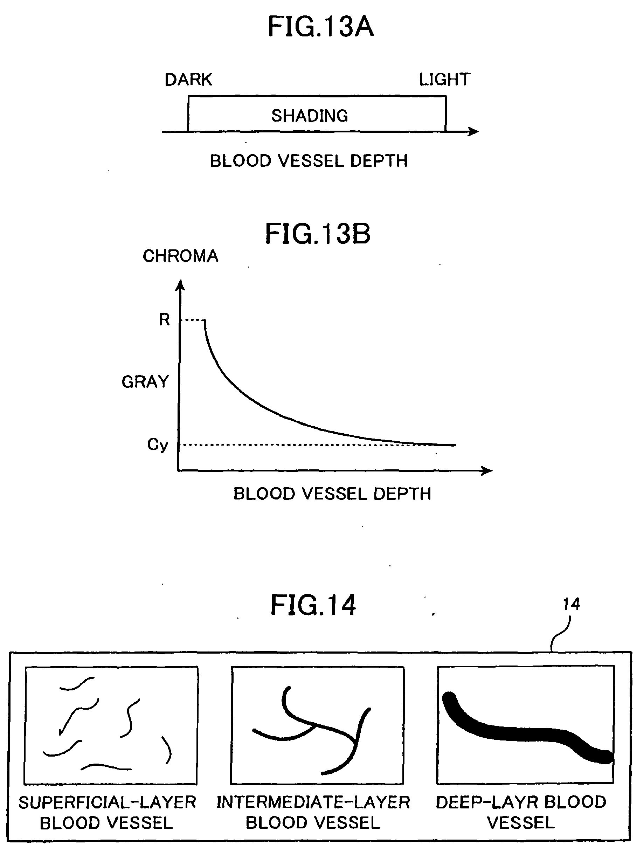

The color information, represented by a color circle in this embodiment, may be represented by shading or luminosity in chromatic color or achromatic color such as black and white as illustrated in Fig. 13. In Fig. 13A, the oxygen saturation level is represented by darker shading (lower luminosity) when the oxygen saturation level is small and represented by lighter shading (higher luminosity) as the oxygen saturation level increases. The oxygen saturation level may alternatively be represented by two-color gradation changing from R to Cy as illustrated in Fig. 13B. The color information illustrated in Fig. 13B is represented by two complementary colors R and Cy; the luminosity changes between R and Cy according to the oxygen saturation level so that the oxygen saturation level is represented by R when it is small, the color information approaching Cy as the oxygen saturation level increases. Because the two-color gradation contains gray as intermediate value, gray is passed as the color information changes between the complementary colors. Visibility tests we conducted showed that the two-color gradation offers a good visibility.

When all the pixels in the blood vessel region have been assigned color information, the oxygen saturation level image producer 64 reads out broadband image data from the frame memory 56 and incorporates the color information in the read-out broadband image data. Thus produced is the oxygen saturation level image data incorporating the oxygen saturation levels of the superficial-layer blood vessel, the intermediate-layer blood vessel, and the deep-layer blood vessel (i.e., representing these oxygen saturation levels in simulated color). The oxygen saturation level image data thus produced is stored again in the frame memory 56. The color information may be incorporated in one of the first to the seventh narrowband image data or in a synthesized image obtained by combining these in lieu of the broadband image data. Alternatively, the broadband image data may be converted into a monochromatic image, and the color information may be incorporated in the monochromatic image. The visibility of the color information increases when incorporated in the first to the seventh narrowband image data or the monochromatic image.

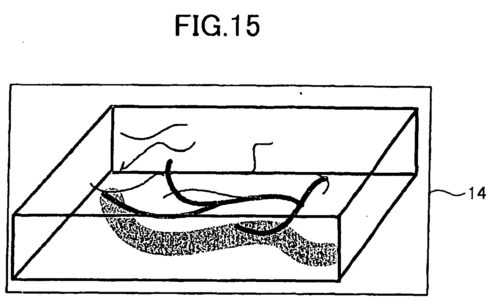

The display control circuit 58 simultaneously displays, in the case of this embodiment, the oxygen saturation level images of the superficial-layer blood vessel, the intermediate-layer blood vessel, and the deep-layer blood vessel on the monitor based on the oxygen saturation level image data stored in the frame memory 56. The oxygen saturation level images of the blood vessels in the respective layers represent a lower oxygen saturation level region (pixel) in cyan (Cy), an intermediate oxygen saturation level region (pixel) in magenta (magenta), and a higher oxygen saturation level region (pixel) in red (R) in simulated color. As illustrated in Fig. 14, the display control circuit 58 can display, for example, three oxygen saturation level images (two-dimensional images) of blood vessels in the respective layers. This mode of display advantageously permits easy observation of two-dimensional distribution of the oxygen saturation level. Further, as illustrate in fig.15 the display control circuit 58 can also display the oxygen saturation level images of blood vessels in the respective layers in simulated three-dimensional images. This mode of display advantageously permits easy observation of variation in oxygen saturation level according to the blood vessel depth. The display mode of the oxygen saturation level images of blood vessels in the respective layers is not limited in any manner; the image selector switch 68 permits selection of the number of images to be displayed on the monitor 14 (one or more), the kinds of images to be displayed simultaneously, the display position, and the like.

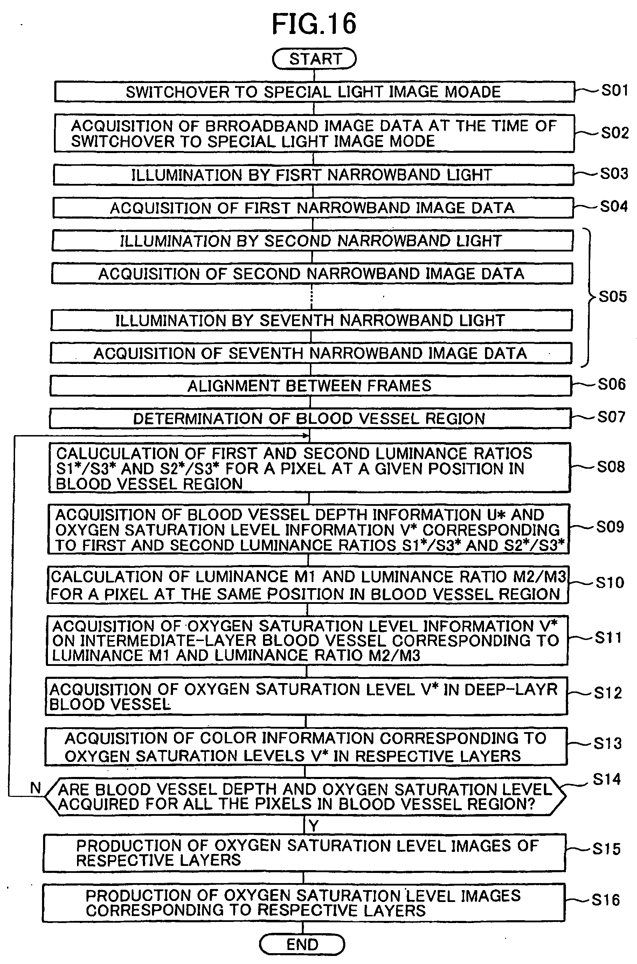

Referring to the flowchart of Fig. 16, we will now describe the operations of the electronic endoscope system 10 for imaging the oxygen saturation levels of a superficial-layer blood vessel, an intermediate-layer blood vessel, and a deep-layerblood vessel.

First, the console 23 is operated to switch from the normal light image mode to the special light image mode (step S01). When the mode is switched to the special light image mode, the broadband image data as of the time when the special light image mode is selected is stored in the frame memory 56 as image data used to produce the oxygen saturation level images of the blood vessels in the respective layers (step S02). The console 23 is also operated to specify a site to be presently observed such as stomach, duodenum, and small intestine. Then, the oxygen saturation level image producer 64 selects one of the color tables 64a to 64c according to the site to be observed. The broadband image data used to produce the oxygen saturation level image may be broadband image data obtained before operating the console.

Upon receiving an illumination stop signal from the controller 59 to shutter actuator 32, the shutter actuator 32 moves the shutter 31 from the retracted position to the inserted position, causing the broadband light BB to stop illuminating the inside of the body cavity. When illumination by the broadband light BB is stopped, the controller 59 sends the light source selector 37 an illumination start instruction. Thereupon, the light source selector 37 turns on the first narrowband light source 91 to illuminate the inside of the body cavity with the first narrowband light N1 (step S03). Upon the narrowband light N1 illuminating the inside of the body cavity, the controller 59 sends the imaging controller 46 an imaging instruction. Thus, imaging is done by illumination with the first narrowband light N1, and the first narrowband imaging signal obtained by the imaging is sent through the AFE 45 to the DSP 55. The DSP 55 produces the first narrowband image data based on the first narrowband imaging signal. The first narrowband image data thus produced is stored in the frame memory 56 (step S04).

When the first narrowband image data has been stored in the frame memory 56, the light source selector 37 switches the light for illuminating the inside of the body cavity from the first narrowband light N1 to the second narrowband light N2 in response to the light source switching instruction from the controller 59. Thereafter, likewise the light for illuminating the inside of the body cavity are sequentially selected to carry out imaging, so that the first to the seventh narrowband image data corresponding to the first to the seventh narrowband light N1 to N7 are produced sequentially and stored in the frame memory 56.

When the first to the seventh narrowband image data have been stored in the frame memory 56, the image alignment processor 65 aligns the images corresponding to the first to the seventh narrowband image data to correct positional shifts between the images acquired in the individual frames caused by the movement of the body tissue or the like. The first to the seventh narrowband image data that have undergone the alignment processing are stored in the frame memory 56 (step S06).

Then, the luminance ratio calculator 60 first determines a blood vessel region containing a blood vessel from the first to the seventh narrowband image data that have undergone the alignment processing (step S07).

Then, the luminance ratio calculator 60 calculates the first luminance ratio S1*/S3* between the first and the third narrowband image data and the second luminance ratio S2*/S3* between the second and the third narrowband image data corresponding to a pixel at the same position in the blood vessel region (step S08). Then, the blood vessel depth-oxygen saturation level calculator 62 obtains the blood vessel depth U* and the oxygen saturation level V* of a superficial-layer blood vessel corresponding to the first and the second luminance ratio S1*/S3* and S2*/S3* based on the correlation in the correlation storage 61 (step S09).

The luminance ratio calculator 60 calculates a luminance M1 of the fourth narrowband image data and a luminance ratio M2/M3 of the second and the fifth narrowband image data for a pixel in the same position in the blood vessel region (step S10). Subsequently, the blood vessel depth-oxygen saturation level calculator 62 determines the oxygen saturation level V* of an intermediate-layer blood vessel corresponding to the luminance M1 and the luminance ratio M2/M3 based on the correlation in the correlation storage 61 (step S11).

Further, as in the case of the intermediate-layer blood vessel, the oxygen saturation level V* for a deep-layer blood vessel is obtained (step S12).

When the oxygen saturation levels of a superficial-layer blood vessel, an intermediate-layer blood vessel, and a deep-layer blood vessel have been obtained, the oxygen saturation level image producer 64 determines color information corresponding to the oxygen saturation level V* for the layers of the superficial-layer blood vessel, the intermediate-layer blood vessel, and the deep-layer blood vessel determined based on the blood vessel depth U* according to one of color tables 64a to 64c selected by the console 23. The color information thus determined is stored in the RAM (not shown) in the processor 12 (step S13).

Upon storage of the color information in the RAM, the above procedure is followed to obtain the blood vessel depth U* and the oxygen saturation level V* for all the pixels in the blood vessel region and determine color information corresponding to the oxygen saturation level V* of the blood vessels in the respective layers (step S14).
Then, when the oxygen saturation level and the corresponding color information have been obtained for all the pixels in the blood vessel region, the oxygen saturation level image producer 64 reads out the broadband image data from the frame memory 56 and incorporates the color information stored in the RAM in the broadband image data to produce the oxygen saturation level image data of the blood vessels in the respective layers. The relative value oxygen saturation level image data of the blood vessels in the respective layers thus produced are stored again in the frame memory 56 (step S15).

Then, the display control circuit 58 reads out the oxygen saturation level image data of the blood vessels in the respective layers from the frame memory 56 and, based on these read-out image data, simultaneously displays the oxygen saturation level images of the superficial-layer blood vessel, the intermediate-layer blood vessel, and the deep-layer blood vessel in a row in juxtaposition, for example, on the monitor 14 as illustrated in Fig. 14. Alternatively, the display control circuit 58 may use the image selector switch 68 to selectively display the oxygen saturation level images of the blood vessels in the respective layers in simulated three-dimensional images as illustrated in Fig. 15 (step S16).

According to this embodiment, the oxygen saturation level images of the blood vessels in the respective layers represent a lower oxygen saturation level region (pixel) in cyan (Cy), an intermediate oxygen saturation level region (pixel) in magenta (magenta), and a higher oxygen saturation level region (pixel) in red (R) in simulated color.

As described above, the electronic endoscope system 10 permits simultaneous acquisition of information on blood vessel depth and oxygen saturation level by switching between wavelengths of illumination light illuminating a subject and simultaneous display of oxygen saturation level images of blood vessels lying in different depths.

Although the above embodiment uses a combination only comprising narrowband light corresponding to the superficial-layer blood vessel, the intermediate-layer blood vessel, and the deep-layer blood vessel to acquire information on the oxygen saturation level, the invention is not limited to this and may use channel information of a part of the color of broadband light in addition to narrowband light. An example of combination is given below for the superficial-layer blood vessel and the deep-layer blood vessel below, respectively.

Superficial-layer blood vessel: narrowband light having central wavelengths of 445 nm and 473 nm and G channel of broadband light

Deep-layer blood vessel: narrowband light having a central wavelength of 800 nm and R channel of broadband light

Specifically, with the superficial-layer blood vessel, the luminance ratio of reflected light obtained by illuminating the subject with narrowband light of 445 nm and 473 nm corresponds to the magnitude of the oxygen saturation level, and the luminance ratio of reflected light of G channel obtained by illuminating the subject with broadband corresponds to the information on the blood vessel density in the subject tissue. With the deep-layer blood vessel, the luminance ratio of reflected light of R channel obtained by illuminating the subject with broadband light corresponds to the magnitude of the oxygen saturation level, and the luminance ratio of reflected light of narrowband light of 800 nm illuminating the subject corresponds to the information on the blood vessel density in the subject tissue. These features are used to obtain the oxygen saturation level from a distribution similar to that illustrated in Fig. 10.

The present invention permits simultaneously acquiring, imaging and displaying distributions of oxygen saturation levels of not only the superficial-layer blood vessel, the intermediate-layer blood vessel, and the deep-layer blood vessel, but a plurality of blood vessels lying in different depths and in any given depth. Further, the number of narrowband light used to acquire the oxygen saturation level information of a plurality of blood vessels lying in different depths is not limited to seven as in the above embodiment.

Still further, the present invention may be applied not only to an insertion type electronic endoscope comprising an insertion section as described above but to a capsule type electronic endoscope comprising an image sensor and the like such as a CCD incorporated in a capsule.

The present invention is basically as described above.

While the invention has been described above in detail, the invention is by no means limited to the above embodiments, and various improvements and modifications may of course be made without departing from the scope of the present invention.

## Claims

1. An electronic endoscope system (10) comprising
a light source device (13) for sequentially emitting light having different wave-length bands,
an electronic endoscope (11) for receiving reflected light from the light emitted from the light source device (13) and sequentially illuminating with different wave-length bands a subject tissue containing a blood vessel inside a body cavity and sequentially outputting an imaging signal corresponding to the wavelength bands of the received light,
an alignment means (65) for aligning images corresponding to imaging signals of light having different wavelength bands outputted from the electronic endoscope, **characterized by**
an image production means (64) for producing oxygen saturation level images each representing a distribution of an oxygen saturation level in a plurality of blood vessels lying in a depth different from the depths corresponding to the other images, from imaging signals of images aligned by the alignment means, and
an image displaying means (14) for simultaneously displaying the oxygen saturation level images of the plurality of blood vessels lying in the different depths producted by the image producing means (64).

2. The electronic endoscope system (10) according to claim 1,
wherein the light source device (13) emits at least two first light and at least one second light as the plurality of light having different wavelength bands, the at least two first light having wavelength bands whereby the magnitude of light absorption reverses between oxygenated hemoglobin and reduced hemoglobin in the blood vessel depending on the oxygen saturation level, the at least one narrowband light having a wavelength band whereby the absorbance coincides.

3. The electronic endoscope system (10) according to claim 2, wherein the light source device (13) emits at least three narrowband light having respective central wavelengths ±10 nm as the at least two first light and the at least one second light.

4. The electronic endoscope system (10) according to claim 3,
wherein the electronic endoscope (11) comprises a color image sensor (44) that converts the received light into the imaging signal through photoelectric conversion, and
wherein the light source device (13) simultaneously emits narrowband light having central wavelengths corresponding to spectral sensitivities of respective color channels of the image sensor (44).

5. The electronic endoscope system (10) according to claim 3,
wherein the light source device (13) emits two narrowband light having the central wavelengths of 405nm and 473nm, respectively as the at least two first light and one narrowband light having a central wavelength of 445nm as the at least one second light for a superficial-layer blood vessel lying in a depth of from 10µm to 100µm, the two narrowband light having the central wavelengths of 473nm and 560nm, respectively as the at least two first light and the one narrowband light having the central wavelength of 532nm as the at least one second light for an intermediate-layer blood vessel lying in the depth of from 100µm to 500µm, and the two narrowband light having the central wavelengths of 560nm and 650nm, respectively as the at least two first light and the one narrowband light having central wavelength of 800nm as the at least one second light for a deeplayer blood vessel lying in the depth of from 500µm to 2000µm.

6. The electronic endoscope system (10) according to claim 3,
wherein the electronic endoscope (11) comprises a monochromatic image sensor (44) that converts the received light into the imaging signal through photoelectric conversion, and
wherein the light source device (13) emits narrowband light having a first central wavelength in such an emission order that the narrowband light having the first central wavelength is emitted between narrowband light having second and third central wavelengths that permit easier acquisition of characteristics information of the subject tissue than does the narrowband light having the first central wavelength.

7. The electronic endoscope system (10) according to claim 3,
wherein the light source device (13) further emits broadband light having a wavelength band of 470 nm to 700 nm in addition to the first and the second light, and
wherein the image producing means (64) produces the oxygen saturation level images from imaging signals of the at least three narrowband light and an imaging signal of at least one color channel of the broadband light that have been aligned by the alignment means (65).

8. The electronic endoscope system (10) according to any of claims 1 to 7, wherein the image display means (14) simultaneously displays the oxygen saturation level images of the plurality of blood vessels lying in the different depths as separate two-dimensional images.

9. The electronic endoscope system (10) according to any of claims 1 to 7, wherein the image display means (14) simultaneously displays the oxygen saturation level images of the plurality of blood vessels lying in the different depths as one three-dimensional image.

10. The electronic endoscope system according to any of claims 1 to 9,
wherein the image producing means produces oxygen saturation level images of a superficial-layer blood vessel, an intermediate-layer blood vessel, and a deep-layer blood vessel, and
wherein the image display means simultaneously displays the oxygen saturation level images of the superficial-layer blood vessel, the intermediate-layer blood vessel, and deep-layer blood vessel.

## Patentansprüche

1. Elektronisches Endoskopsystem (10), umfassend:
eine Lichtquelleneinrichtung (13) zum sequentiellen Emittieren von Licht unterschiedlicher Wellenlängenbänder,
ein elektronisches Endoskop (11) zum Empfangen reflektierten Lichts von dem von der Lichtquelleneinrichtung (13) emittierten Licht und zum sequentiellen Beleuchten eines Patientengewebes, das im Inneren eines Körperhohlraums ein Blutgefäß enthält, mit unterschiedlichen Wellenlängenbändern, und zum sequentiellen Ausgeben eines Abbildungssignals entsprechend den Wellenlängenbändern des empfangenen Lichts,
eine Ausrichteinrichtung (65) zum Ausrichten von Bildern entsprechend den Abbildungssignalen von Licht mit unterschiedlichen Wellenlängenbändern, die von dem elektronischen Endoskop ausgegeben werden, **gekennzeichnet durch**
eine Bilderzeugungseinrichtung (64) zum Erzeugen von Sauerstoffsättigungswertbildern, die jeweils eine Verteilung eines Sauerstoffsättigungswerts in mehreren Blutgefäßen repräsentieren, welche in einer Tiefe liegen, die verschieden ist von den Tiefen, die den anderen Bildern entsprechen, aus Bildsignalen von **durch** die Ausrichteinrichtung ausgerichteten Bildern, und
eine Bildanzeigeeinrichtung (14) zum gleichzeitigen Anzeigen der Sauerstoffsättigungswertbilder der mehreren Blutgefäße, die sich in den verschiedenen Tiefen befinden, erzeugt von der Bilderzeugungseinrichtung (64).

2. Elektronisches Endoskopsystem (10) nach Anspruch 1,
bei dem die Lichtquelleneinrichtung (13) als die mehreren Lichter unterschiedlicher Wellenlängenbänder mindestens zwei erste Lichter und mindestens ein zweites Licht emittiert, von denen die zumindest zwei ersten Lichter Wellenlängenbänder aufweisen, bei denen die Lichtmengenabsorption sich zwischen oxidiertem Hemoglobin und reduziertem Hemoglobin in dem Blutgefäß abhängig von dem Sauerstoffsättigungswert umkehrt, während das mindestens eine schmalbandige Licht ein Wellenlängenband aufweist, bei dem die Absorption übereinstimmt.

3. Elektronisches Endoskopsystem (10) nach Anspruch 2, bei dem die Lichtquelleneinrichtung (13) mindestens drei schmalbandige Lichter mit entsprechenden Mittenwellenlängen ± 10 nm als die mindestens zwei ersten Lichter und das mindestens eine zweite Licht emittiert.

4. Elektronisches Endoskopsystem (10) nach Anspruch 3,
bei dem das elektronische Endoskop (11) einen Farbbildsensor (44) aufweist, der das empfangene Licht durch photoelektrische Umwandlung in das Abbildungssignal umwandelt, und
bei dem die Lichtquelleneinrichtung (13) gleichzeitig schmalbandiges Licht mit Mittenwellenlängen entsprechend den Spektralempfindlichkeiten jeweiliger Farbkanäle des Bildsensors (44) emittiert.

5. Elektronisches Endoskopsystem (10) nach Anspruch 3,
bei dem die Lichtquelleneinrichtung (13) zwei schmalbandige Lichter mit den Mittenwellenlängen von 405 nm bzw. 473 nm als die mindestens zwei ersten Lichter, und ein schmalbandiges Licht mit einer Mittenwellenlänge von 445 nm als das mindestens eine zweite Licht für ein Oberflächenschicht-Blutgefäß, das in einer Tiefe von 10 µm bis 100 µm liegt, emittiert, die zwei schmalbandigen Lichter mit Mittenwellenlängen von 473 nm bzw. 560 nm als die mindestens zwei ersten Lichter und das eine schmalbandige Licht mit der Wellenlänge von 532 nm als das mindestens eine zweite Licht für ein Zwischenschicht-Blutgefäß, das in einer Tiefe von 100 µm bis 500 µm liegt, emittiert, und die zwei schmalbandigen Lichter mit den Mittenwellenlängen von 560 nm bzw. 650 nm als die mindestens zwei ersten Lichter und das eine schmalbandige Licht mit einer Mittenwellenlänge von 800 nm als das mindestens eine zweite Licht für ein Tiefenschicht-Blutgefäß, das in der Tiefe von 500 µm bis 2000 µm liegt, emittiert.

6. Elektronisches Endoskopsystem (10) nach Anspruch 3, bei dem das elektronische Endoskop (11) einen monochromatischen Bildsensor (44) aufweist, der das empfangene Licht durch photoelektrische Umwandlung in das Abbildungssignal umwandelt, und
bei dem die Lichquelleneinrichtung (13) schmalbandiges Licht mit einer ersten Mittenwellenlänge in einer derartigen Emissionsreihenfolge emittiert, dass das schmalbandige Licht mit der ersten Mittenwellenlänge zwischen dem schmalbandigen Licht mit zweiter und dritter Mittenwellenlänge emittiert wird, die ein leichteres Erfassen charakteristischer Information des Patientengewebes ermöglichen, als dies bei dem schmalbandigen Licht mit der ersten Mittenwellenlänge der Fall ist.

7. Elektronisches Endoskopsystem (10) nach Anspruch 3,
bei dem die Lichtquelleneinrichtung (13) weiterhin breitbandiges Licht mit einem Wellenlängenband von 470 nm bis 700 nm zusätzlich zu dem ersten und dem zweiten Licht emittiert, und
die Bilderzeugungseinrichtung (64) die Sauerstoffsättigungswertbilder aus Abbildungssignalen der mindestens drei schmalbandigen Lichter und einem Abbildungssignal mindestens eines Farbkanals des breitbandigen Lichts erzeugt, die durch die Ausrichteinrichtung (65) ausgerichtet wurden.

8. Elektronisches Endoskopsystem (10) nach einem der Ansprüche 1 bis 7, bei dem die Bildanzeigeeinrichtung (14) gleichzeitig die Sauerstoffsättigungswertbilder der mehreren Blutgefäße, die in unterschiedlichen Tiefen liegen, als getrennte zweidimensionale Bilder anzeigt.

9. Elektronisches Endoskopsystem (10) nach einem der Ansprüche 1 bis 7, bei dem die Bildanzeigeeinrichtung (14) gleichzeitig die Sauerstoffsättigungswertbilder der mehreren in unterschiedlichen Tiefen liegenden Blutgefäße als ein dreidimensionales Bild darstellt.

10. Elektronisches Endoskopsystem (10) nach einem der Ansprüche 1 bis 9,
bei dem die Bilderzeugungseinrichtung Sauerstoffsättigungswertbilder eines Oberflächenschicht-Blutgefäßes, eines Zwischenschicht-Blutgefäßes und eines Tiefenschicht-Blutgefäßes erzeugt, und
die Bildanzeigeeinrichtung gleichzeitig die Sauerstoffsättigungswertbilder des Oberflächenschicht-Blutgefäßes, des Zwischenschicht-Blutgefäßes und des Tiefenschicht-Blutgefäßes anzeigt.

## Revendications

1. Système d'endoscope électronique (10), comprenant :
un dispositif de source lumineuse (13) destiné à émettre de manière séquentielle des lumières présentant des bandes de longueurs d'ondes différentes ;
un endoscope électronique (11) destiné à recevoir une lumière réfléchie à partir de la lumière émise à partir du dispositif de source lumineuse (13) et illuminant de manière séquentielle avec des bandes de longueurs d'ondes différentes un tissu de sujet contenant un vaisseau sanguin à l'intérieur d'une cavité corporelle, et à délivrer de manière séquentielle un signal d'imagerie correspondant aux bandes de longueurs d'ondes de la lumière reçue ;
un moyen d'alignement (65) destiné à aligner des images correspondant à des signaux d'imagerie de lumière présentant des bandes de longueurs d'ondes différentes, délivrés à partir de l'endoscope électronique, **caractérisé par**
un moyen de production d'images (64) destiné à produire des images de niveau de saturation en oxygène, chacune représentant une distribution d'un niveau de saturation en oxygène dans une pluralité de vaisseaux sanguins se trouvant à une profondeur différente de la profondeur correspondant des autres images, à partir de signaux d'imagerie d'images alignées par le moyen d'alignement, et
un moyen d'affichage d'images (14) destiné à afficher simultanément des images de niveau de saturation en oxygène de la pluralité de vaisseaux sanguins se trouvant à des profondeurs différentes, produites par le moyen de production d'images (64).

2. Système d'endoscope électronique (10) selon la revendication 1,
dans lequel le dispositif de source lumineuse (13) émet au moins deux premières lumières et au moins une seconde lumière pour la pluralité de lumières présentant différentes bandes de longueurs d'ondes différentes, les au moins deux premières lumières présentant des bandes de longueurs d'ondes où la grandeur de l'absorption lumineuse s'inverse entre l'hémoglobine oxygénée et l'hémoglobine réduite dans le vaisseau sanguin en fonction du niveau de saturation en oxygène, la au moins une lumière à bande étroite présentant une bande de longueurs d'ondes où l'absorbance coïncide.

3. Système d'endoscope électronique (10) selon la revendication 2,
dans lequel le dispositif de source lumineuse (13) émet au moins trois lumières à bande étroite présentant des longueurs d'ondes centrales respectives ±10 nm pour les au moins deux premières lumières et la au moins une seconde lumière.

4. Système d'endoscope électronique (10) selon la revendication 3,
dans lequel l'endoscope électronique (11) comprend un capteur d'image couleur (44) qui convertit la lumière reçue en le signal d'imagerie par le biais d'une conversion photoélectrique, et
dans lequel le dispositif de source lumineuse (13) émet simultanément une lumière à bande étroite présentant des longueurs d'ondes centrales correspondant à des sensibilités spectrales de canaux de couleur respectifs du capteur d'image (44).

5. Système d'endoscope électronique (10) selon la revendication 3,
dans lequel le dispositif de source lumineuse (13) émet deux lumières à bande étroite présentant les longueurs d'ondes centrales de 405 nm et de 473 nm, respectivement pour les au moins deux premières lumières et une lumière à bande étroite présentant une longueur d'onde centrale de 445 nm pour la au moins une seconde lumière pour un vaisseau sanguin de couche superficielle se trouvant à un profondeur allant de 10 µm à 100 µm, les deux lumières à bande étroite présentant les longueurs d'ondes centrales de 473 nm et 560 nm, respectivement pour les au moins deux premières lumières et la une lumière à bande étroite présentant la longueur d'onde centrale de 532 nm pour la au moins une seconde lumière pour un vaisseau sanguin de couche intermédiaire se trouvant à une profondeur allant de 100 µm à 500 µm, et les deux lumières à bande étroite présentant les longueurs d'ondes centrales de 560 nm et de 650 nm, respectivement pour les au moins deux premières lumières et la une lumière à bande étroite présentant une longueur d'onde centrale de 800 nm pour la au moins une seconde lumière pour un vaisseau sanguin de couche profonde se trouvant à une profondeur allant de 500 µm à 2000 µm.

6. Système d'endoscope électronique (10) selon la revendication 3,
dans lequel l'endoscope électronique (11) comprend un capteur d'image monochrome (44) qui convertit la lumière reçue en le signal d'imagerie par le biais d'une conversion photoélectrique, et
dans lequel le dispositif de source lumineuse (13) émet une lumière à bande étroite présentant une première longueur d'onde centrale dans un ordre d'émission tel que la lumière à bande étroite présentant la première longueur d'onde centrale est émise entre une lumière à bande étroite présentant des deuxième et troisième longueurs d'ondes centrales qui permettent une acquisition d'informations de caractéristiques du tissu de sujet plus facile que dans le cas de la lumière à bande étroite présentant la première longueur d'onde centrale.

7. Système d'endoscope électronique (10) selon la revendication 3,
dans lequel le dispositif de source lumineuse (13) émet en outre une lumière à large bande présentant une bande de longueurs d'ondes allant de 470 nm à 700 nm en plus des première et seconde lumières, et
dans lequel le moyen de production d'images (64) produit des images de niveau de saturation en oxygène à partir de signaux d'imagerie des au moins trois lumières à bande étroite et un signal d'imagerie d'au moins un canal de couleur de la lumière à large bande, qui ont été alignées par le moyen d'alignement (65).

8. Système d'endoscope électronique (10) selon l'une quelconque des revendications 1 à 7, dans lequel le moyen d'affichage d'images (14) affiche simultanément les images de niveau de saturation en oxygène de la pluralité de vaisseaux sanguins se trouvant à des profondeurs différentes sous la forme d'images bidimensionnelles séparées.

9. Système d'endoscope électronique (10) selon l'une quelconque des revendications 1 à 7, dans lequel le moyen d'affichage d'images (14) affiche simultanément les images de niveau de saturation en oxygène de la pluralité de vaisseaux sanguins se trouvant à des profondeurs différentes sous la forme d'une image tridimensionnelle.

10. Système d'endoscope électronique (10) selon l'une quelconque des revendications 1 à 9,
dans lequel le moyen de production d'images produit des images de niveau de saturation en oxygène d'un vaisseau sanguin de couche superficielle, d'un vaisseau sanguin de couche intermédiaire et d'un vaisseau sanguin de couche profonde, et
dans lequel le moyen d'affichage d'images affiche simultanément des images de niveau de saturation en oxygène du vaisseau sanguin de couche superficielle, du vaisseau sanguin de couche intermédiaire et du vaisseau sanguin de couche profonde.
